# EUROPÄISCHE PATENTANMELDUNG

(11) **EP 1 726 285 A1**
(43) Veröffentlichungstag der Anmeldung: **29.11.2006**
(21) Anmeldenummer: 05104386.7
(22) Anmeldetag: 24.05.2005
(51) Int. Cl.: A61J 1/06, B65D 1/09

(54) **Behälter für die Abgabe eines Medikaments und zugehörige Verabreichungsvorrichtung**

(71) Anmelder: VIFOR (INTERNATIONAL) AG, 9001 St Gallen (CH)
(72) Erfinder: Weibel, Ludwig, 9104 Waldstatt (CH); Weibel-Furer, Dominique, 9104 Waldstatt (CH)
(74) Vertreter: Gille Hrabal Struck Neidlein Prop Roos

(57) **Zusammenfassung**

Die vorliegende Erfindung betrifft einen Behälter (1, 1', 1", 1''') für die Aufnahme und Abgabe eines Medikaments, insbesondere parenteralen Medikaments, wobei der Behälter (1, 1', 1", 1''') durch eine bis auf eine Öffnung (7) zur Abgabe des Medikaments verschlossene Umhüllung (6, 6') aufweist, mit Mitteln zur Verbindung (4) der Öffnung mit einer Verabreichungsvorrichtung, wobei der Behälter (1, 1', 1", 1''') so gestaltet ist, dass unter Änderung von wenigstens einem Bereich der Umhüllung (6, 6') eine Abgabe des Medikaments erfolgt. Die Umhüllung (6, 6') ist ferner einstückig ausgebildet. Die Erfindung betrifft ferner die zugehörige Verabreichungsvorrichtung.

## Beschreibung

Die vorliegende Erfindung betrifft einen Behälter für die Aufnahme eines Medikaments und dessen Abgabe in eine Verabreichungsvorrichtung, dessen Herstellung und Verwendung sowie die zugehörige Verabreichungsvorrichtung. Bei den Medikamenten handelt es sich beispielsweise um parenteral zu verabreichende Medikamente, aber auch orale oder topische Medikamente. Der erfindungsgemäße Behälter weist eine besonders vorteilhafte Ausgestaltung der Umhüllung auf.

Bei der parenteralen, d.h. den Darm eines Säugers umgehenden Verabreichung von Medikamenten geschieht dies meist durch Injektion oder Infusion. Unter einer Injektion versteht man die Verabreichung eines flüssigen, sterilen Medikamentes mittels Spritze und Hohlnadel direkt in das Gewebe oder Gefäßsystem unter Umgehung des Magen-Darm-Traktes. Bei der Infusion handelt es sich um langsames, meist tropfenweises Einfließen größerer (arzneimittelhaltiger) Flüssigkeitsmengen in den Körper.

Medikamente, beispielsweise parenteral zu verabreichende Medikamente, werden im Allgemeinen nach ihrer Zubereitung in einen Behälter abgefüllt, der eine oder mehrere Portionen/Dosen aufnehmen kann. Dieser Behälter wird als Primärpackung bezeichnet.

Als Primärpackung sind Glas-Ampullen bekannt die mit einer speziellen Ampullensäge zu öffnen sind oder die zum Zwecke der Öffnung mit einer Sollbruchstelle versehen sind. Der Inhalt aus der Ampulle muss in einen für die Verabreichung geeigneten Behälter, wie z.B. eine Spritze, transferiert werden. Ferner muss meist noch Restflüssigkeit in der Ampulle verbleiben, da es ansonsten bei dem Aufziehen der Spritze nachteilig zum Ansaugen von Luft kommen kann. Darüber hinaus ist ein späterer Verschluss der Ampulle mit der darin enthaltenden Restflüssigkeit praktisch unmöglich.

Als weiterer Behälter ist aus dem Stand der Technik eine sogenannte Stechampulle (Vial) bekannt. Diese wird mit einer Spritze an einer dafür vorgesehenen Stelle eingestochen, und die Spritze wird dann mit der im Behälter befindlichen Flüssigkeit aufgezogen. Dieses mit diesem Behälter verbundene Verfahren ist sehr aufwendig, da die Ampulle nicht unmittelbar zur Verabreichung verwendet werden kann, sondern der Inhalt aus der Ampulle in einen für die Verabreichung geeigneten Behälter, wie die zuvor genannte Spritze, transferiert werden muss. Das Öffnen oder Anstechen von Ampullen und ein anschließendes notwendiges Transferieren in einen für die Verabreichung geeigneten Behälter erweist sich z.B. in Notsituationen bei denen die Verabreichung unter Zeitdruck zu erfolgen hat, als nachteilig.

Heute wird auch unmittelbar in eine Spritze abgefüllt, die zur Verabreichung mit einer aufgesteckten oder eingeklebten Kanüle versehen sind. Durch Pressen des im Spritzenbehälter beweglich gelagerten Kolbens wird die Flüssigkeit aus dem Behälter durch die Kanüle in den Verabreichungsort injiziert. Die Spritze weist den Nachteil auf, dass sie aufgrund des mehrteiligen Aufbaus vergleichsweise teuer ist. Zur Aufrechterhaltung der Funktionsfähigkeit auch nach längerer Lagerdauer sind Kolben und Spritzenbehälter mit Beschichtungen, bspw. mit Silikon, versehen. Damit wird eine Substanz zusammen mit dem Medikament gelagert und verabreicht, die mit der eigentlichen Wirkung des Medikaments nichts zu tun hat und sich z.B. bei Medikamenten mit einem hohen pH Wert sogar nachteilig auswirken kann.

Nebst den parenteralen Medikamenten, welche i.a. in Ampullen, Vials oder Fertigspritzen, sowie in Infusionsflaschen bzw. -beuteln abgefüllt werden, werden auch teilweise nicht parenteral verabreichte Medikamente in Ampullen oder Vials aufbewahrt, da sie vor der Verabreichung z.B. mit Wasser gemischt werden müssen.

Vor dem Hintergrund der oben beschriebenen Nachteile ist es daher Aufgabe der vorliegenden Erfindung einen Behälter für die Aufnahme und Abgabe eines Medikaments bzw. eine zugehörige Verabreichungsvorrichtung bereitzustellen, welche jeweils die Verabreichung eines Medikaments erleichtern und die vergleichsweise einfach aufgebaut sind.

Diese Aufgabe wird durch den Behälter des Anspruchs 1, sowie die Vorrichtung des Anspruchs 16 gelöst. Vorteilhafte Ausgestaltungen ergeben sich aus den Unteransprüchen. Ein vorteilhaftes Herstellungsverfahren sowie eine vorteilhafte Verwendung sind Gegenstand der nebengeordneten Ansprüche.

Der erfindungsgemäße Behälter zur Aufnahme und Abgabe eines Medikaments ist mit einer bis auf eine Öffnung zur Abgabe des Medikaments verschlossenen und einstückigen Umhüllung versehen. Der Behälter ist ferner derart ausgestaltet, dass unter Änderung der ganzen oder von einem Bereich der Umhüllung eine Abgabe des Medikaments aus der Öffnung erfolgt.
Das Medikament liegt beispielsweise in einer Lösung oder sonstigen Flüssigkeit vor, wobei die Viskosität unterschiedlich gewählt sein kann. Beispielsweise handelt es sich um ein parenteral, oral oder topisch zu verabeichendes Medikament. Die Öffnung kann in einer Ausgestaltung mit Verschlussmitteln verschlossen sein, um das im Behälter aufgenommene Medikament vor Kontamination zu schützen. Die Verschlussmittel umfassen beispielsweise Schraub- oder Steckkappen und an die Umhüllung angeschweißte und mit Sollbruchstelle versehene Abschnitte.

Durch die Mittel zur Verbindung der Öffnung mit einer Verabreichungsvorrichtung, beispielsweise einer Infusions- oder Injektionsvorrichtung, kann das Medikament aus dem erfindungsgemäßen Behälter direkt verabreicht werden. Beispielsweise ist der Behälter mit einer Kanüle verbunden.

Erfindungsgemäß ist die Änderung der Umhüllung mit der Abgabe des Medikaments verbunden. Beispielsweise ist die Umhüllung aufgrund der Materialwahl und insbesondere bei einer vergleichsweise großvolumigen Umhüllung kollabierend ausgestaltet, das heißt, dass bei der Abgabe des Medikaments mittels Kapillarkräfte und / oder aufgrund der Schwerkraftwirkung aufgrund des sich im Behälter ausbildenden Unterdrucks es zur wenigstens teilweisen Kollabierung der Umhüllung und somit Änderung der Umhüllung kommt.

Die Einstückigkeit der Umhüllung sorgt einerseits für eine preiswerte Herstellung der Vorrichtung. Ferner erfolgt die Verabreichung besonders einfach, steril, schnell und störunanfällig. Aufgrund der Einstückigkeit ist beispielsweise gegenüber einer Konstruktion mit beweglichem Kolben die Anzahl der produktberührenden Materialien reduziert, so dass die Kompatibilität mit dem Medikament vorteilhaft und vergleichsweise einfach sichergestellt werden kann.

Bei einer weiteren vorteilhaften Ausgestaltung des erfindungsgemäßen Behälters ist dieser so ausgestaltet, dass die Abgabe des Medikaments durch Druck auf wenigstens einen Außenbereich der Umhüllung erreicht oder wenigstens ausgelöst wird. Beispielsweise ist die Umhüllung derart gestaltet, dass durch einen Daumendruck auf den zugehörigen Außenbereich der Umhüllung eine Abgabe des Medikaments bewirkt wird. Dadurch wird eine besonders reproduzierbare, dosierte Abgabe des Medikaments, das beispielsweise in flüssiger Form mit unterschiedlichster Viskosität vorliegt, ermöglicht. Beispielsweise wird der für eine Injektion erforderliche Injektionsdruck, durch den auf die Umhüllung einwirkenden Druck generiert, zusätzliche Mittel zur Druckerzeugung, beispielsweise eine Pumpe, können somit vorteilhaft entfallen. Beispielsweise ist der Behälter röhrenförmig ausgebildet. Ein Druck auf die röhrenförmige Umhüllung löst eine Volumenverkleinerung aus. Die zugehörige Volumenverkleinerung bewirkt eine Verdrängung des sich im Behälter aufgenommenen Medikaments aus dem Behälter und damit ein Austreten und damit eine Abgabe über die Öffnung. Je nach gewählter Härte der Umhüllung kann die Charakteristik des aufzubringenden Druckes vorteilhaft variiert werden.

Der Behälter ist in einer weiteren vorteilhaften Ausführungsform so ausgestaltet, dass nach der Auslösung der Abgabe durch den Druck die Abgabe des Medikaments selbständig fortgesetzt wird. Beispielsweise wird dies durch eine Ausgestaltung erreicht, bei der ein relativ harter und elastischer Umhüllungsabschnitt bei Erreichen einer gewissen Außenkrümmung nach dem Knackfrosch-Prinzip federartig in die entgegengesetzte, in das Behälter Innere weisende Krümmung überspringt und so aufgrund der Federcharakteristik des Umhüllungsabschnitts ein genau definiertes Medikamentenvolumen abgibt. Dadurch wird einerseits eine selbständige und andererseits wohl dosierte Abgabe erreicht.

Der Behälter weist gemäß einer weiteren vorteilhaften Ausführungsform eine tubenförmige Umhüllung auf. "Tubenförmig" im Sinne der Erfindung ist so zu verstehen, dass der Behälter im Wesentlichen infolge der Verschweißung zu seinem der Öffnung gegenüberliegendem Ende spitz zuläuft. Durch diese Art der Ausgestaltung wird eine besonders leichte Entleerung des Behälters und damit Abgabe des Medikaments erreicht. Beispielsweise kann durch Streichbewegung von dem spitzen Ende der Tube eine besonders effektive, d.h. schnelle und weitgehend restlose Entleerung des Behälters erreicht werden, insbesondere wenn das Medikament im Vergleich zu wässrigen Lösungen eine hohe Viskosität aufweist. Eine Tube wird beispielsweise durch eine Röhre erzeugt, bei der ein Ende durch flaches Verschweißen oder Falzen jeweils in Abhängigkeit des verwendeten Materials verschlossen ist. Der vor dem Verschweißen oder Falzen bestehende Zugang ins Innere des tubenförmigen Behälters dient in einer Ausgestaltung vorteilhaft zudem als Zuführmöglichkeit für die Befüllung mit dem Medikament, das nachfolgend im Behälter aufgenommen wird und über die erfindungsgemäß vorgesehene Öffnung gegebenenfalls abgegeben wird.

Gemäß einer weiteren vorteilhaften Ausführungsform sind die Mittel zur Verbindung zwischen der Öffnung und der Verabreichungsvorrichtung lösbar gestaltet. Die Mittel zur lösbaren Verbindung weisen beispielsweise eine Ausgestaltung auf, die es ermöglicht, den Behälter an eine dem Patienten bereits eingesetzte Injektions-/Infusionskanüle anzuschließen und wieder zu lösen. Ferner ist beispielsweise zum Anschluss ein mit Außengewinde versehenen Fortsatz am Behälter vorgesehen, in dem die Öffnung konzentrisch angeordnet ist. Die Verabreichungsvorrichtung, wie eine Injektions- bzw. Infusionsvorrichtung weist beispielsweise eine mit dem Fortsatz zusammenwirkende und diesen aufnehmende Vertiefung auf. Durch das in der Vertiefung vorgesehene und mit dem Außengewinde zusammenwirkende Innengewinde wird eine lösbare aber gleichzeitig flüssigkeitsdichte Verbindung zwischen dem Behälter und der Verabreichungsvorrichtung erreicht. Die ausgestaltungsgemäße Lösbarkeit der Verbindung zwischen Behälter und Infusions- bzw. Injektionsmitteln stellt sicher, dass ein Behälter leicht durch einen befüllten Behälter ausgetauscht werden kann, wodurch die Flexibilität und Schnelligkeit in der Verabreichung und damit die Benutzerfreundlichkeit erhöht wird. Ferner kann der zum Transport und der Lagerung verwendete Behälter gleichzeitig zur Verabreichung des Medikaments verwendet werden. Dadurch entfällt vorteilhaft ein Transferiervorgang von einem Transportbehälter in einen für die Verabreichung bestimmten Behälter.

Bei einer weiteren vorteilhaften Ausführungsform umfassen die Mittel zur Verbindung der Öffnung mit der Verabreichungsvorrichtung ein Arretiermittel gegen ein unbeabsichtigtes Lösen der Verbindung auf. Beispielsweise ist eine Schraubvorrichtung mit einer Einrastfunktion versehen, die die Schraubverbindung im eingeschraubten Zustand durch ein einrastendes Federelement zusätzlich sichert. Ein Lösen ist erst nach Überwinden dieser, mit der Einrastung verbundenen, Federwirkung möglich und verhindert so weitgehend ein unbeabsichtigtes Lösen der Verbindung.

Gemäß einer weiteren vorteilhaften Ausführungsform des erfindungsgemäßen Behälters weisen die Mittel zur lösbaren Verbindung eine konische verlaufende Fläche auf. Diese wirkt beispielsweise mit einer weiteren konischen Fläche an der Verabreichungsvorrichtung, wie einer Injektions- oder Infusionsvorrichtung zusammen. Dadurch wird eine einfache und gleichzeitig ausreichend dichte Verbindung erreicht. Beispielsweise ist der Behälter mit einem Fortsatz mit konischer Außenfläche oder mit einer Vertiefung mit konischer Innenfläche versehen.

Gemäß einer Ausführungsform handelt es sich um einen Luer-Anschluss (Luer cone oder Luer lock). Im Allgemeinen handelt es sich, um einen als "male" bezeichneten Anschluss. Durch Vorsehen eines solchen Anschlusses am Behälter werden bei der Verabreichung des Medikaments mehrere Arbeitsschritte eingespart werden. Ein Luer"female" Anschluss ist vorgesehen, wenn im Behälter ein Medikament aufbewahrt wird, welches zuerst rekonstituiert werden muss.

Die Mittel zur lösbaren Verbindung sind gemäß einer weiteren vorteilhaften Ausgestaltung einstückig mit dem Behälter ausgebildet. Dadurch kommt vorteilhaft bei der Abgabe des Medikaments kein weiteres Material mit dem Medikament in Berührung. Der Kontakt des Medikaments beschränkt sich während der Aufbewahrung und Abgabe auf ein Material bzw. eine Materialzusammensetzung. So kann besonders einfach sichergestellt werden, dass der Kontakt mit Materialien des Behälters sich nicht nachteilig, beispielsweise pH-Wert ändernd, auf das im Behälter enthaltende Medikament auswirkt. Ferner kann so der Behälter besonders einfach, kostengünstig und schnell hergestellt werden, was in herstellungstechnischer Hinsicht von Vorteil ist.

Die Einstückigkeit wird beispielsweise in einem einzigen Herstellungsschritt oder wie folgt erreicht: in einem ersten Schritt der Herstellung werden zwei Stücke hergestellt, welche anschließend verschweißt oder zusammengesteckt werden. Der Abfüller wird dann mit einem einstückigen Teil beliefert, um dies mit dem Medikament zu befüllen. Eine Zusammenführung zweier Teile ist von Vorteil, wenn auf vorhandene Spritzformen zurückgegriffen wird, bei einem Herstellverfahren, wo Kunststoff- oder Laminatschläuche, die den eigentlichen Behälter bilden, an ein Kopfteil mit den Verbindungsmitteln angeschweißt werden oder wenn die Verbindungsmittel eine besondere Komplexität aufweisen.

Bei einer weiteren vorteilhaften Ausgestaltung weist der Behälter ein Volumen auf von weniger als 5ml auf. Ein Volumen von weniger als 5ml erweist sich als besonders vorteilhaft, da ein Behälter, insbesondere eine Tube durch einfachen Druck mit dem Daumen in einem Zug effizient und gleichmäßig entleerbar ist. Es hat sich gezeigt, dass dadurch die Handhabung des Behälters und damit die Medikamentverabreichung besonders einfach und effizient ist.

Der Behälter besteht in einer weiteren vorteilhaften Ausführungsform aus Kunststoff, insbesondere Polypropylen, PE, wie LDPE oder PET. Weiter verwendbar sind PVC, PVF etc. Heute werden auch neue, verschweißbare Kunststoffe entwickelt, welche für Medikamente vorteilhaft einsetzbar sind. Die erfindungsgemäße Kunststoffauswahl ist nur insoweit eingeschränkt, dass dadurch die Herstellbarkeit des erfindungsgemäßen Behälters gewährleistet ist. Dadurch kann der Behälter preiswert hergestellt werden. Ferner kann aufgrund der vergleichsweise niedrigen Schweiß- und Schmelztemperatur das Verschließen des Behälters nach erfolgter Abfüllung besonders medikamentschonend erfolgen. Darüber hinaus kann so leicht die Chargen- und / oder Verfallsinformation leicht auf die Außenfläche des Behälters eingeprägt werden. Diese Beschriftungen sind so von hoher Dauer.

Der Behälter besteht bei einer weiteren vorteilhaften Ausführungsform aus einem Laminat. Dadurch bestehen weitere Möglichkeiten der Kompatibilität. Beispielsweise kann in den Behältermantel eine Aluminiumfolie eingelegt werden, welche selbst nicht produktberührend ist, jedoch eine erhöhte Sauerstoffbarriere darstellt und so die Produktstabilität günstig beeinflusst. Weiter können vorgedruckte Etiketten, welche ihrerseits wiederum Barrierefunktion aufweisen können, in den Mantel eingelegt werden (in mold Labelling). Dadurch entfällt sogar das aufkleben von Etiketten und es wird kein Kleber notwendig, welcher durch Diffusion ins Produkt gelangen könnte.

Gemäß einer weiteren vorteilhaften Ausführungsform ist der Behälter wenigstens teilweise transparent ausgebildet. Dadurch kann vorteilhaft der Füllstand bzw. die Entleerung kontrolliert werden.

Der Behälter ist gemäß einer weiteren vorteilhaften Ausgestaltung beschichtet. Beispielsweise ist die mit dem Medikament in Berührung stehende innere Fläche der Umhüllung mit einer chemisch neutralen Beschichtung, wie Siliziumoxid beschichtet. Dadurch wird erreicht, dass der Behälter aufgrund der Beschichtung, in dieser Hinsicht glasähnliche Eigenschaft erreicht, ohne die mit Glas verbundenen Nachteile aufzuweisen: beispielsweise Verletzungsgefahr beim Öffnen und Entsorgen von Glasampullen usw..

Die Umhüllung des Behälters ist bei einer weiteren vorteilhaften Ausführungsform wenigstens teilweise elastisch ausgebildet. Dadurch wird erreicht, dass sich bei Wegnahme des Drucks auf die Umhüllung diese zurückbilden kann und beispielsweise in mehreren gleichartigen hintereinander ausgeführten Bewegungen der Behälter stoßweise entleert werden kann. Dadurch kann die Entleerung besonders einfach erfolgen.

Die Mittel zur Verbindung können ferner gemäß einer weiteren Ausführungsform mit einem dafür bestimmten Verschluss zusammenwirken, so dass ein besonders leichtes Verschließen des erfindungsgemäßen Behälters, beispielsweise im Gegensatz zu den eingangs beschriebenen Ampullen, erreicht wird. Der Verschluss ist beispielsweise ein separates Kunststoffspritzgussteil, das auf den zuvor beschriebenen Fortsatz aufgeschraubt oder aufgesteckt wird. In einer weiteren Ausführungsform ist der Verschluss einstückig mit der Umhüllung verbunden und wird zusammen mit dem Behälter in einem Verarbeitungsschritt hergestellt. Dadurch kann der Behälter samt Verschluss einfach und kostengünstig hergestellt werden.

Die Erfindung betrifft ferner eine Vorrichtung zur Verabreichung eines Medikaments, insbesondere eines parenteralen Medikaments, mit einem Behälter gemäß einer der vorhergehenden Ansprüche. Die Mittel zur Verabreichung eines Medikaments sind dabei mittels der Verbindungsmittel über die Öffnung mit dem Behälter verbunden. Die Verabreichungsvorrichtung sind gemäß einer Ausführungsform im Bereich der Öffnung in die Umhüllung eingesetzt oder eingeklebt. Gemäß einer weiteren Ausführungsform ist die Umhüllung einstückig mit einem nadelförmigen Ansatz um die Öffnung versehen. Die jeweiligen Vorteile des zuvor beschriebenen Behälters betreffen gleichfalls die erfindungsgemäße Vorrichtung.

Die erfindungsgemäße Vorrichtung weist in einer weiteren Ausführungsform Mittel zur Infusion oder Injektion auf. Beispielsweise handelt es sich um eine Kanüle. Die Injektion erfolgt je nach Verabreichungsort intracutan, subcutan, intramuskulär, intravenös, intraarteriell, intrakardial, intraartikular, intrathekal oder intralumbal.

Das erfindungsgemäße Verfahren zur Herstellung des Behälters weist einen Schritt auf bei dem in einem Kunststoffspritzguss-, Kunststoffspritzblas- oder einem Kunststoffextrusionsblasschritt der Behälter hergestellt wird. Dadurch kann der Behälter preiswert, gegebenenfalls zusammen mit dem Verschluss und / oder den Mitteln zur lösbaren Verbindung mit den Verabreichungsvorrichtungen hergestellt werden. In einem weiteren Herstellverfahren werden Mantel und Kopfteil separat hergestellt und miteinander verschweißt. Ferner kann die für den Abfüller und Anwender vorteilhafte Einstückigkeit dadurch erreicht werden, dass zwei Teile vom Hersteller separat hergestellt, aber bereits von diesem zu einem Stück zusammengefügt (gesteckt und anschließend z.B. mittels Ultraschall verschweißt) werden.

Die Erfindung betrifft ferner eine Verwendung des Behälters in einer der zuvor beschriebenen Ausführungsformen zum Aufziehen einer Spritze. Dadurch kann vorteilhaft eine Spritze besonders einfach und effizient aufgezogen werden. In einer weiteren Ausführungsform wird die Spritze mit mehreren Behältern flüssigkeitsleitend verbunden, um beim Aufziehen der Spritze einerseits ein Mischen, Lösen der in den Behältern vorhandenen Medikamente bzw. Lösungsmittel zu erreichen und diese gleichzeitig in das Spritzenvolumen zu transferieren.

Ferner betrifft die Erfindung eine Verwendung des Behälters zur Aufnahme eines Granulates oder eines Lyophilisates, welche zur Verabreichung mit einem Lösungsmittel rekonstituiert oder aufgelöst werden müssen. Das Lösungsmittel, z.B. Wasser, kann vorteilhaft direkt mit einem analogen, erfindungsgemäßen Behälter, das Gegenstück zum Anschluss enthaltend oder mittels eines Adapters in den ersten Behälter gegeben werden. Alternativ kann eine Spritze oder etwas Entsprechendes benutzt werden. Die Mischung und Applikation erfolgt besonders einfach dank der flexiblen Umhüllung.

### Zu den Figuren:

Die Figuren 1a bis 1c zeigen Ausführungsbeispiele des erfindungsgemäßen Behälters.

Die Figuren 2a und 2b zeigen eine Form des erfindungsgemäßen Behälters, wobei Bereiche der Umhüllung selbst kollabierend ausgeführt sind.

Figur 3 zeigt Adapter zur flüssigkeitsleitenden Verbindung mehrerer erfindungsgemäßer Behälter.

Figur 4 veranschaulicht das Aufziehen einer Spritze mit dem erfindungsgemäßen Behälter.

Figur 5 zeigt die Einbringung eines Medikaments aus dem erfindungsgemäßen Behälter in eine Spritze, in der ein Lösungsmittel oder ein weiteres Medikament enthalten ist, um diese in der Spritze zu weiteren Verabreichung zu vermischen bzw. untereinander zu lösen.

Figur 6 zeigt die Einbringung eines Medikaments aus zwei erfindungsgemäßen Behältern in eine Spritze. Dabei können die Inhalte in den zwei erfindungsgemäßen Behältern zuerst miteinander gemischt und aus demjenigen mit dem Luer male Anschluss direkt appliziert oder eben wie dargestellt zur Verabreichung in eine Spritze aufgezogen werden Figur 6 zeigt insbesondere die beliebige Flexibilität des Systems mit den erfindungsgemäßen Behältern.

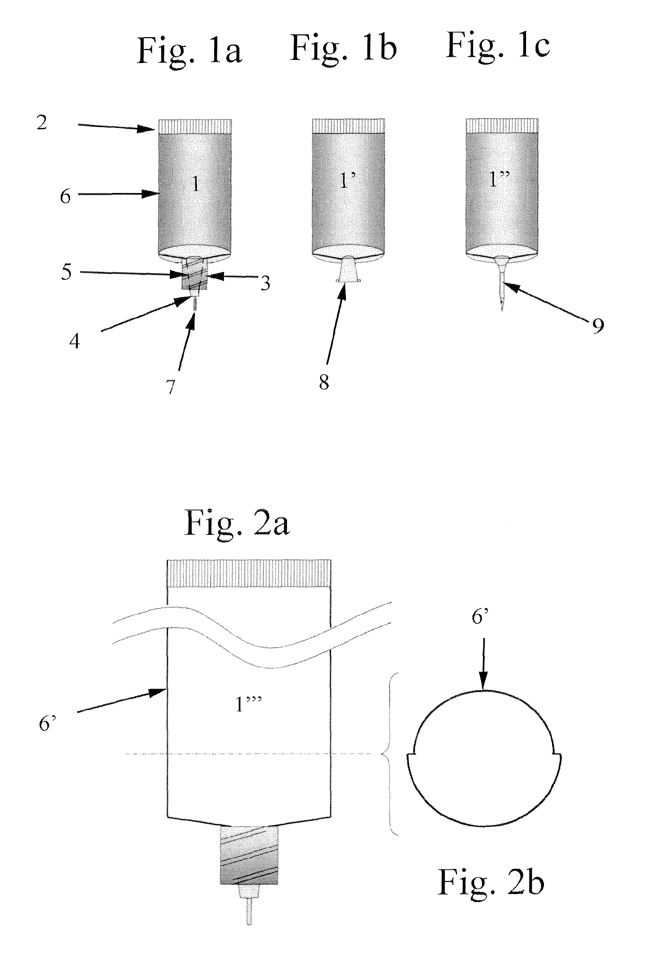

Figur 1a zeigt eine erste Ausführungsform des erfindungsgemäßen Behälters 1 mit einer tubenförmigen, einstückigen Umhüllung 6. In der Umhüllung 6 sind eine, ein (i.a. parenterales) Medikament beinhaltende, Flüssigkeit (nicht dargestellt) oder ein auflösbarer Feststoff enthalten. Das (i.a. parenterale) Medikament bzw. die Flüssigkeit wird über die Öffnung 4 aus dem Behälter 1 abgegeben, indem im Bereich der 1 auf die Umhüllung 6 beispielsweise mit den Fingern gepresst wird. Die Umhüllung 6 ist auf der der Öffnung 4 gegenüber liegenden Seite mit einer eben verlaufenden Verschweißung 2 luft- und flüssigkeitsdicht verschlossen, wodurch sich die Tubenform ergibt. Die Mittel zur Verbindung der Öffnung 4 des Behälters mit einer nicht gezeigten Verabreichungsvorrichtung umfassen einen an der Umhüllung ausgebildeten konischen Fortsatz 5. Dessen Verlauf ist in der Fig. 1a gestrichelt angedeutet, da er teilweise von einer mit Innengewinde versehenen Hülse 3 umgeben ist, die ebenfalls Teil der Verbindungsmittel ist. Der konische Fortsatz kann ohne die Hülse vorgesehen sein und bildet somit einen sogenannten Luer male -Anschluss. Ist die Hülse mit Innengewinde vorgesehen ergibt sich ein Aufbau, der einem sogenannten Luer lock Anschluss entspricht. Der konisch verlaufende Fortsatz 3 als Teil der Mittel zur lösbaren Verbindung wirkt beispielsweise mit einer konisch nach innen verlaufenden Ausnehmung einer Verabreichungsvorrichtung oder einem Adapter (Dreifachadapter), einer Luftfalle, einem Luftfilter, einem Ventil, einer Kanüle, einem Infusionsbehälter oder sonstigen Infusions- oder Injektionsmitteln zusammen, um diese einerseits flüssigkeitsleitend andererseits dicht und lösbar zu verbinden.

Die Öffnung 4 ist an der Austrittsstelle mit einer Sollbruchstelle verschlossen, wobei ein beliebig ausgeformtes Bruchhilfestück 7 angegossen wird. Weiter kann die Öffnung mit einer in diesem Fall auch direkt angegossenen Membran erreicht werden, welcher z.B. mit einer Spritzenspitze (mit oder ohne Nadel) durchstochen werden kann.

Fig. 1b zeigt eine weitere Ausführungsform 1' des erfindungsgemäßen, tubenförmigen Behälters. Dieser unterscheidet sich vor allem von der Ausführungsform 1 durch die andersartige Gestaltung der Umhüllung im Bereich der Öffnung. Die Mittel zur Verbindung umfassen eine konische geformte Ausnehmung 8. In die Ausnehmung ist ein konisch geformter Fortsatz einer weiteren Vorrichtung einbringbar, um diese flüssigkeitsleitend mit dem Behälter 1' zu verbinden.

Fig. 1c zeigt eine weitere Ausführungsform 1" des erfindungsgemäßen, tubenförmigen Behälters. Dieser unterscheidet sich vor allem von der Ausführungsform 1 durch die andersartige Gestaltung der Umhüllung im Bereich der Öffnung. Der Behälter ist mit einem hohinadelfömigen Fortsatz 9 versehen, der zur Injektion des Medikaments oder Einbringung desselben z.B. in einen Infusionsbeutel dient.

Die Figuren 2a und 2b zeigen eine weitere Ausführungsform 1"' des erfindungsgemäßen Behälters. Dieser ist mit einer Umhüllung 6' versehen, die bei der Abgabe des Medikaments das Knackfrosch-Prinzip ausnutzt. In Figur 2b ist die Umhüllung 6' im Schnitt dargestellt. Die in der Figur gezeigte obere Hälfte der Umhüllung springt nach innen elastisch über, nachdem nach anfänglichem äußeren Druck eine gewisse Krümmung überschritten ist, diese nach innen gerichtete Federwirkung bewirkt einen Verdrängungseffekt auf das in der Umhüllung 6' befindliche Medikament und führt zu einer Abgabe des Medikaments über die Öffnung. Aufgrund der vorgegebenen Federwirkung wird reproduzierbar selbständig nach dem ersten, auslösenden Druck auf die Umhüllung 6' eine Abgabe bewirkt. Somit wird immer die gleiche Flüssigkeitsmenge ausgestoßen. Im Querschnitt ist ersichtlich, dass die obere Hälfte der Umhüllung nach Druck in die andere Hälfte eingepasst und so auch nicht mehr in die ursprüngliche Form zurückschnellen kann.

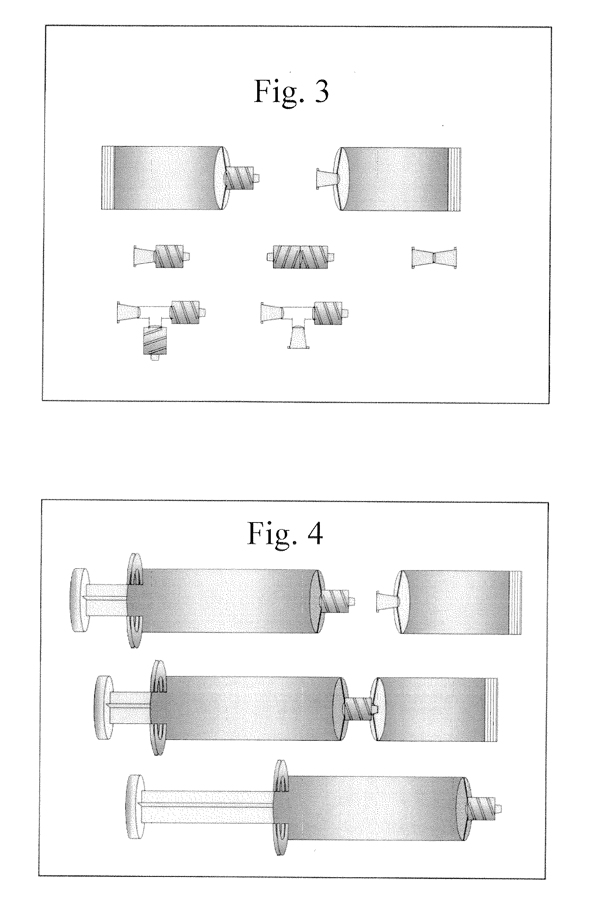

Figur 3 zeigt unterschiedliche Adapter zur Verbindung der erfindungsgemäßen Behälter untereinander oder zur Verbindung der Behälter mit einer Luftfalle, einem Luftfilter, einem Ventil, einer Kanüle oder sonstigen Infusions- oder Injektionsmitteln, wie beispielsweise einer Injektionsspritze.

Figur 4 illustriert das Aufziehen einer Spritze mit dem im erfindungsgemäßen Behälter enthaltenden, bspw. parenteralen, Medikament. Die zeitliche Abfolge der Schritte entspricht der Anordnung der Figuren von oben nach unten. Eine Spritze wird mit ihrem konischen Fortsatz in die konische Ausnehmung des erfindungsgemäßen Behälters eingebracht. Die Spritze wird danach aufgezogen und der Inhalt gelangt über die Öffnung des Behälters unter Änderung der Umhüllung des Behälters in das Spritzenvolumen.

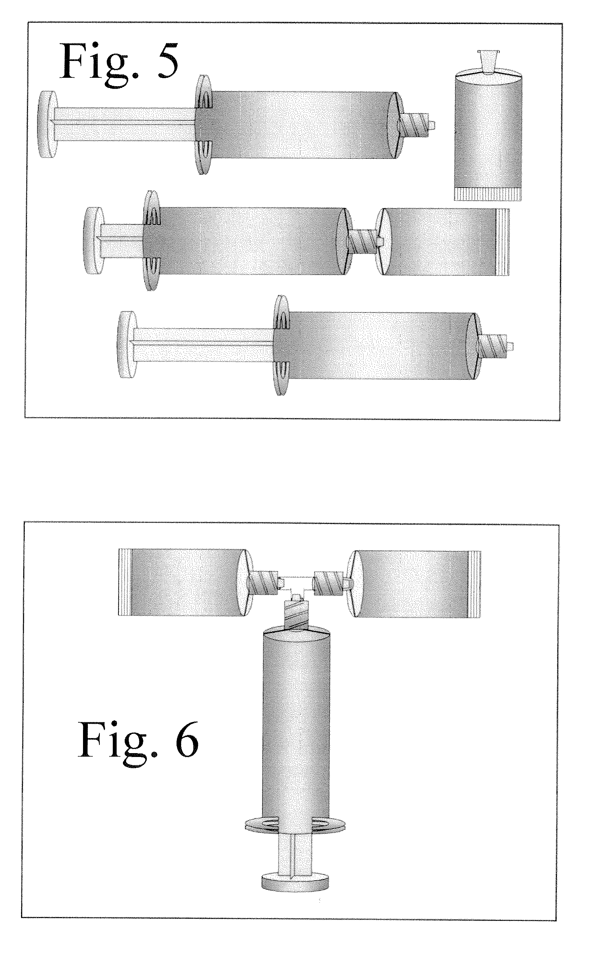

Figur 5 zeigt eine Anwendung bei der ein in dem erfindungsgemäßen Behälter enthaltendes Lyophilisat mit einem Lsm, das sich in der Spritze befindet, vermischt wird. Das Lsm wird mittels der Spritze in den Behälter gespritzt. Vermischt sich mit dem Lyophilisat im Behälter und wird dann gemäß einem Anwendungsbeispiel zurück in die Spritze befördert, um damit verabreicht zu werden.

Figur 6 zeigt die Einbringung eines Medikaments aus zwei erfindungsgemäßen Behältern in eine Spritze, um diese in der Spritze zur weiteren Verabreichung zu vermischen bzw. untereinander zu lösen. Beispielsweise ist in einem tubenförmigen Behälter ein Lyophilisat und in dem weiteren Behälter ein Lsm enthalten, die vermischt werden und beim Aufziehen der Spritze in das Spritzenvolumen gelangen.

## Patentansprüche

1. Behälter (1, 1', 1", 1"') für die Aufnahme und Abgabe eines Medikaments, insbesondere parenteralen Medikaments, wobei der Behälter (1, 1', 1", 1"') durch eine bis auf eine Öffnung (7) zur Abgabe des Medikaments verschlossene Umhüllung (6, 6') aufweist, mit Mitteln zur Verbindung (4) der Öffnung mit einer Verabreichungsvorrichtung, wobei der Behälter (1, 1', 1", 1"') so gestaltet ist, dass unter Änderung von wenigstens einem Bereich der Umhüllung (6, 6') eine Abgabe des Medikaments erfolgt, **dadurch gekennzeichnet, dass** die Umhüllung (6, 6') einstückig ausgebildet ist.

2. Behälter (1, 1', 1", 1"') gemäß dem vorhergehenden Anspruch, wobei der Behälter (1, 1', 1", 1"') so ausgestaltet ist, dass die Abgabe des Medikaments durch Druck auf wenigstens einen Außenbereich der Umhüllung (6) erreicht oder wenigstens ausgelöst wird.

3. Behälter (1, 1', 1", 1"') gemäß dem vorhergehenden Anspruch, wobei der Behälter (1, 1', 1", 1"') so ausgestaltet ist, dass nach der Auslösung der Abgabe des Medikaments die Abgabe des Medikaments selbständig fortgesetzt wird.

4. Behälter (1, 1', 1", 1"') gemäß einem der vorhergehenden Ansprüche,
wobei der Behälter (1, 1', 1", 1"') eine tubenförmige Umhüllung (6, 6') aufweist.

5. Behälter (1, 1', 1", 1"') gemäß einem der vorhergehenden Ansprüche,
wobei die Mittel zur Verbindung (3, 8) so ausgestaltet sind, dass die Verbindung lösbar ist.

6. Behälter (1, 1', 1", 1"') gemäß dem vorhergehenden Anspruch, wobei die Mittel zur Verbindung (3, 8) Arretiermittel gegen unbeabsichtigtes Lösen umfassen.

7. Behälter (1, 1', 1", 1"') gemäß einem der vorhergehenden Ansprüche,
wobei die Mittel zur Verbindung (3, 8) eine konisch verlaufende Fläche umfassen.

8. Behälter (1, 1', 1", 1"') gemäß einem der vorhergehenden Ansprüche,
wobei die Mittel zur Verbindung (3, 8) einstückig mit dem Behälter (1, 1', 1", 1"') ausgebildet sind.

9. Behälter (1, 1', 1", 1"') gemäß einem der vorhergehenden Ansprüche,
wobei der Behälter (1, 1', 1", 1"') ein Volumen von weniger als 5ml aufweist.

10. Behälter (1, 1', 1", 1"') gemäß einem der vorhergehenden Ansprüche,
wobei der Behälter (1, 1', 1", 1"') aus Kunststoff, insbesondere Polypropylen, PE, wie LDPE oder PET, besteht.

11. Behälter (1, 1', 1", 1"') gemäß einem der vorhergehenden Ansprüche,
wobei der Behälter (1, 1', 1", 1"') ein Laminat aufweist.

12. Behälter (1, 1', 1", 1"') gemäß einem der vorhergehenden Ansprüche,
wobei der Behälter (1, 1', 1", 1"') wenigstens teilweise transparent ausgebildet ist.

13. Behälter (1, 1', 1", 1"') gemäß einem der vorhergehenden Ansprüche,
wobei der Behälter (1, 1', 1", 1"') teilweise beschichtet ist.

14. Behälter (1, 1', 1", 1"') gemäß einem der vorhergehenden Ansprüche,
wobei die Umhüllung (6, 6') des Behälters (1, 1', 1", 1"') wenigstens teilweise elastisch ausgebildet ist.

15. Behälter (1, 1', 1", 1"') gemäß einem der vorhergehenden Ansprüche,
wobei die Mittel zur Verbindung (3, 8) so gestaltet sind, dass sie mit einem Verschlusselement (7) zusammenwirken, um die Öffnung (4) des Behälters zu verschließen.

16. Vorrichtung zur Verabreichung eines Medikaments, insbesondere eines parenteralen Medikaments, mit einem Behälter gemäß einer der vorhergehenden Ansprüche.

17. Vorrichtung zur Verabreichung eines Medikaments gemäß dem vorhergehenden Anspruch, das Mittel zur Infusion oder Injektion umfasst.

18. Verfahren zur Herstellung des Behälters (1, 1', 1", 1"') gemäß einem der vorhergehenden Ansprüche 1 - 13, das einen Spritzguss-, Spritzblas- oder einen Extrusionsblasschritt umfasst.

19. Verwendung des Behälters (1, 1', 1", 1"') gemäß einem der vorhergehenden Ansprüche 1 - 13 zum Aufziehen einer Spritze.
